# EUROPÄISCHE PATENTANMELDUNG

(11) **EP 1 588 690 A1**
(43) Veröffentlichungstag der Anmeldung: **26.10.2005**
(21) Anmeldenummer: 05006521.8
(22) Anmeldetag: 24.03.2005
(51) Int. Cl.: A61K 7/06

(54) **Verzögerung oder Verhinderung von unerwünschtem Haarwuchs**

(30) Priorität: 20.04.2004 DE 102004018954
(71) Anmelder: elements medical GmbH, 60529 Frankfurt/Main (DE)
(72) Erfinder: Riegel, Erwin, 65779 Kelkheim (DE)
(74) Vertreter: Luderschmidt, Schüler & Partner GbR

(57) **Zusammenfassung**

Mischung und Verfahren zur Verzögerung oder Verhinderung von unerwünschtem Haarwuchs, wobei die Mischung a) Äpfelsäure, b) einen Elektrolyten und c) ein in der Kosmetik akzeptables, auf Wasser basierendes Mittel, das ein filmbildendes Mittel einschließt, aufweist.

## Beschreibung

Die vorliegende Erfindung bezieht sich auf eine Mischung zur äußerlichen Anwendung, um unerwünschten Haarwuchs zu verhindern oder zu verzögern.

Vor allem aus kosmetischen Gesichtspunkten ist es wünschenswert, unerwünschten Haarwuchs am menschlichen Körper zu hemmen. Dies trifft besonders auf Teile des menschlichen Körpers wie Beine, Achseln und das Gesicht zu. Dabei soll Haarwuchs möglichst dauerhaft gehemmt werden, damit die meist schmerzhaften und reizenden mechanischen Verfahren, wie die Rasur oder das Epilieren, nicht oder nur begrenzt angewandt werden müssen.

Neben mechanischen Verfahren zum entfernen unerwünschten Haarwuchses existieren chemische Verfahren. Dabei werden diese Produkte, die Thioglycollate als wirksame Bestandteile enthalten, auf die Hautpartie aufgetragen. Die Wirkstoffe reduzieren meist bei hohem pH-Wert die Disulfidbrücken des Haares, wodurch dieses geschwächt wird und meist direkt unter der Hautoberfläche abbricht.

Ein Nachteil dieses Verfahrens ist, dass der Harrwuchs nicht beeinflusst wird. Dadurch, dass das Haar an der Hautoberfläche bricht, wächst es innerhalb kurzer Zeit wieder nach, wodurch eine weitere Anwendung nötig wird. Dauerhafte Anwendung von Thioglycollaten hat allerdings häufig Hautreizungen zur Folge.

Eine Mischung sowie ein Verfahren zur dauerhaften Haarentfernung ist in WO 98/52515 beschrieben. Durch Auftragen von Citronensäure, einem Elektrolyten und einem filmbildenden Material auf Wasserbasis ist es möglich im Anwendungszeitraum ungewollten Haarwuchs zu reduzieren oder zu verhindern.

Ein Nachteil dieses Verfahrens ist, dass auch bei Anwendung des in WO 98/5251 offenbarten Präparates Hautreizungen auftreten.

Ein weiterer Nachteil ist, dass der Erfolg der Anwendung erst nach einer Anwendungsdauer von ca. 6 bis 8 Wochen zu beobachten ist. Diese lange Zeitspanne führt in der Praxis dazu, dass in vielen Fällen die Anwendung abgebrochen wird.

Daher ist es Aufgabe der Erfindung, ein Mittel zur Haarentfernung zu liefern, das die oben genannten Nachteile nicht zeigt.

Speziell ist es Aufgabe der Erfindung, ein Mittel zur dauerhaften Verlangsamung und/oder Verhinderung von unerwünschtem Harrwuchs zu liefern, das zu weniger Hautreizungen führt.

Eine weitere Aufgabe der Erfindung ist es, die lange Zeitspanne bis zum Auftreten von Erfolgen der Anwendung zu verkürzen.

Überraschenderweise wurde gefunden, dass eine Mischung nach Anspruch 1 ein weiteres Mittel zur dauerhaften Verlangsamung und/oder Verhinderung von unerwünschtem Haarwuchs liefert.

Des Weiteren wurde gefunden, dass durch die Anwendung eines Mittels gemäß Anspruch 1 die Hautverträglichkeit bei dauerhafter Anwendung verbessert werden kann.

Auch wurde überraschenderweise gefunden, dass durch Zusatz von Antiandrogenen und Aminosäuren, wie Prolin oder Hydroxyprolin, vorzugsweise in Form von Pflanzenextrakten, die Anwendungszeit bis zum Auftreten erster Erfolge der Anwendung verkürzt werden kann, ohne dass durch diesen Zusatz die Hautverträglichkeit der Mischung zusätzlich beeinträchtigt wird.

Detaillierte Beschreibung der Erfindung

Eine Mischung zur Verlangsamung und / oder Verhinderung von Haarwuchs gemäß dieser Erfindung weist, bezogen auf das Gesamtgewicht der Mischung: (a) 5 bis 30 Gewichtsprozent Äpfelsäure; (b) 1 bis 20 Gewichtsprozent eines Elektrolyten, und (c) 50 bis 94 Gewichtsprozent eines in der Kosmetik akzeptablen, auf Wasser basierenden Mittels, das, bezogen auf das Gesamtgewicht des auf Wasser basierenden Mittels, 0,05 bis 40 Gewichtsprozent eines filmbildenden Mittels aufweist, auf.

Vorzugsweise liegt die Konzentration an Äpfelsäure im Bereich von 5 bis 20 Gewichtsprozent, bezogen auf das Gesamtgewicht der Mischung.

In einer bevorzugten Ausführungsform ist der Elektrolyt ausgewählt aus einem oder mehreren Mitgliedern der Gruppe, bestehend aus wasserlöslichen Salzen von Alkali- und/oder Erdalkalimetallen. Besonders bevorzugt wird als Elektrolyt Natriumchlorid oder Totes Meer Salz verwendet.

Der Anteil des Elektrolyten liegt im Bereich von 1 bis 20 Gewichtsprozent, bezogen auf das Gesamtgewicht der Mischung, vorzugsweise im Bereich von 5 bis 10 Gewichtsprozent.

Das in der Kosmetik akzeptable, auf Wasser basierende Mittel dient als Verdünnungsmittel, Dispersionsmittel und/oder Träger für die anderen Inhaltsstoffe der Mischung, um die Verteilung dieser Inhaltsstoffe bei der Auftragung auf die Haut zu erleichtern. Das Mittel ist ausgewählt aus der Gruppe, bestehend aus Emulsionen, Mikroemulsionen und wässrigen Lösungen eines filmbildenden Mittels.

Das filmbildende Mittel kann aus einer Vielzahl von Substanzen ausgewählt werden, einschließlich Substanzen, ausgewählt aus der Gruppe, bestehend aus Hauterweichern, Emulgatoren, oberflächenaktiven Substanzen, Gelen, Feuchthaltemitteln, Verdickungsmitteln, Pudern und anderen Proteinlösungen.

Diese filmbildenden Mittel können alleine oder als Mischungen mehrerer filmbildender Mittel verwendet werden. Der Anteil des filmbildenden Mittels liegt vorzugsweise im Bereich von 0,05 bis 40 Gewichtsprozent, bezogen auf das Gesamtgewicht des in der Kosmetik akzeptablen, auf Wasser basierenden Mittels.

Stoffe, die als Hauterweicher verwendet werden können, sind dem Fachmann bekann. Diese schließen Mineralöle, Fettalkohole, Alkylester, Silikone oder Silikonderivate ein. Bevorzugt verwendete Fettalkohole sind Stearylalkohol und Hexadecylalkohol. Bevorzugt verwendete Alkylester sind Octylpalmitat, Decyloleat und Isopropylmyristat. Bevorzugt verwendete Silicone und Siliconderivate sind Phenyldimethicon, Dimethicon und Simethicon. Diese Stoffe können alleine oder als Mischungen verwendet werden. Es wird allerdings betont, dass die vorliegende Erfindung nicht auf die oben genannten Hauterweicher beschränkt ist.

Stoffe, die als Emulgatoren oder oberflächenaktive Substanzen verwendet werden können, sind dem Fachmann bekann. Deren Wahl entscheidet meist über das spätere Vorliegen der Emulsion als Wasser in Öl-Emulsion oder Öl in Wasser-Emulsion. Dabei können ein oder mehrere Emulgatoren oder oberflächenaktive Substanzen verwendet werden. Emulsionen enthalten 0,5 bis 15 Gewichtsprozent Emulgatoren, bezogen auf das Gesamtgewicht des in der Kosmetik akzeptablen, auf Wasser basierenden Mittels, bevorzugt 3 bis 10 Gewichtsprozent.

Mikroemulsionen enthalten bevorzugt 0,5 bis 35 Gewichtsprozent, besonders bevorzugt von 15 bis 28 Gewichtsprozent oberflächenaktive Substanzen, bezogen auf das Gesamtgewicht des in der Kosmetik akzeptablen, auf Wasser basierenden Mittels.

Bevorzugt verwendete Emulgatoren schließen Glycerylstearat und PEG-100-Stearat sowie Mischungen daraus (Cithrol GMS AS NA (Croda ES 1862)), Methylglucosesesquistearat (Tegocare PS (Goldschmidt)), hydroxyliertes Rizinusöl und Glycerinsorbitanoleostearat sowie Mischungen daraus ein. Die vorliegende Erfindung soll allerdings nicht auf die genannten Emulgatoren beschränkt bleiben.

Bevorzugte oberflächenaktive Substanzen sind ausgewählt aus der Gruppe, bestehend aus Ceteareth-20 (Emulgin B 2 (Cognis)), Oleth-20 (Volpo N 20 (Croda)), Laureth-4 (DOW CORNING 2-1691 (Dow Corning) und TEGO Alkynol L4 (Goldschmidt)) Lanolinalkohol und Mineralöl, PEG 7 Glycerincocoat (Cetiol HE (Cognis)) und Glycereth-20 (Liponic EG1 (Lipo Australian agents Bronson and Jacobs P/L)) sowie Mischungen daraus, allerdings soll die vorliegende Erfindung nicht auf die genannten oberflächenaktiven Substanzen beschränkt bleiben.

Eine Vielzahl an Gelen, einschließlich Sclerotium Gum (Amigel), kann in dem in der Kosmetik akzeptablen, auf Wasser basierenden Mittel verwendet werden, um Polymerfilme zu bilden. Dabei wird das Gel in einer Konzentration von 0,1 bis 2 Gewichtsprozent, bezogen auf das Gewicht des in der Kosmetik akzeptablen, auf Wasser basierenden Mittels, verwendet.

Weiter kann eine Vielzahl von Pudern im Rahmen der vorliegenden Erfindung verwendet werden. Bevorzugt werden in der vorliegenden Erfindung Puder, wie Lehmpuder, Silicapuder, Talkpuder und Stärkepuder, verwendet.

Auch kann eine Vielzahl an Proteinlösungen, einschließlich Bier, kann als filmbildendes Mittel verwendet werden.

Die Mischung der vorliegenden Erfindung kann ein wasserlösliches Befeuchtungsmittel enthalten. Solche Befeuchtungsmittel sind dem Fachmann bekannt. Bevorzugt werden Befeuchtungsmittel, wie Glycerin und Polyethylenglycole in der vorliegenden Erfindung verwendet, allerdings soll die vorliegende Erfindung nicht auf die genannten Befeuchtungsmittel beschränkt bleiben. Bevorzugt werden Befeuchtungsmittel in einer Konzentration von 1 bis 5 Gewichtsprozent, bezogen auf das Gewicht des in der Kosmetik akzeptablen, auf Wasser basierenden Mittels verwendet.

Weiter kann eine Mischung entsprechend der vorliegenden Erfindung ein oder mehrere Antischweißmittel und/oder deodorierende Substanzen aufweisen. Dadurch können Mischungen nach der vorliegenden Erfindung regelmäßig unter der Achsel angewandt werden, um nicht nur den Haarwuchs sondern auch das Schwitzen und/oder schlechten Geruch zu reduzieren oder zu verhindern. Als Antischweißmitteln werden vorzugsweise astringente Metallsalze, wie Aluminiumchlorid, Aluminiumchlorhydrat und Zirkonchlorhydrat, verwendet. Ein geeignetes Deodorant ist beispielsweise Triclosan (Irgasan DP 300)

Weiter ist bevorzugt, dass das auf Wasser basierende Mittel zusätzlich ein Mittel zur Verzögerung des Wachstums von Körperhaaren aufweist.

Dieses kann antiandrogene Substanzen, wie Phytosterole und makrozyklische Tannine, und/oder Aminosäuren aufweisen, welche die Ausbildung der helicalen Struktur des Keratins des Körperhaares destabilisieren und/oder zerstören, wie beispielsweise Prolin oder Hydroxyprolin.

Vorzugsweise wird das Mittel zur Verzögerung des Wachstums von Körperhaaren aus Pflanzen extrahiert. Besonders bevorzugt ist die Verwendung von Extrakten aus der Sägepalme, aus Kürbiskernen und dem Weidenröschen als Mittel zur Verzögerung des Wachstums von Körperhaaren.

Vorzugsweise weist eine Mischung gemäß der vorliegenden Erfindung einen pH-Wert auf, der nicht kleiner ist als 3,5, um die Haut nicht übermäßig zu beanspruchen. Des Weiteren ist der pH-Wert der Mischung vorzugsweise nicht größer als 4,5, um die Wirksamkeit der Mischung nicht zu beeinträchtigen. Die Einstellung des pH-Wertes erfolgt dabei vorzugsweise durch Zugabe einer Base, besonders bevorzugt durch Zugabe von Natriumhydroxid, zu der Mischung.

Diese erfindungsgemäße Mischung liegt dabei bevorzugt als Creme, Lotion, Gel oder Spray vor.

Eine erfindungsgemäße Mischung wird bevorzugt an ausgewählten Körperstellen, wie Gesicht, Nacken, Oberlippe, Kinn, Beinen, Armen, Rumpf und den Achseln zur Verzögerung oder zur Verhinderung von unerwünschtem Haarwuchs verwendet.

Dabei trägt man die Mischung auf die betreffende Körperstelle auf. Um den Haarwuchs effizient zu verhindern wird die Mischung pro Anwendung zumindest für 30 Minuten, bevorzugt für 4 bis 12 Stunden, auf der Hautpartie belassen.

Die Zeitspane pro Anwendung beträgt bei vorheriger Haarentfernung an der betreffenden Stelle mindestens 5 Minuten, bevorzugt 4 Stunden bis 12 Stunden, um den Haarwuchs effizient zu verhindern.

Die Mischung wird vorzugsweise 3 mal pro Woche, besonders bevorzugt täglich und ganz besonders bevorzugt zwei mal täglich auf die entsprechende Hautpartie aufgetragen. Vorzugsweise wird die Mischung, wenn sie einmal auf die Haut aufgetragen ist, nicht speziell entfernt, kann aber im Rahmen der täglichen Wäsche entfernt werden.

Die Verringerung des Haarwachstums, wenn die erfindungsgemäße Mischung regelmäßig angewandt wird, kann quantitativ gemessen werden. Dazu wird in bestimmten Zeitintervallen das nachgewachsene Haar entfernt und gewogen.

Bei der Anwendung des erfindungsgemäßen Mittels ist allerdings zu beachten, dass die Zeitspanne, innerhalb derer eine Verminderung des Haarwuchses auftritt, variieren kann und abhängig ist von der individuellen Dicke der Haare, der genetischen Veranlagung und der individuellen Häufigkeit, in der die betreffende Hautpartie gewaschen wird.

### Beispiel:

| Rohstoff | Anteil (Gew.%) |
|---|---|
| Salz aus dem Toten Meer | 6,20 |
| Entmineralisiertes Wasser | 66,84 |
| Äpfelsäure | 10,06 |
| Chitrol GMS AS NA | 0,5 |
| 1,2-Propylenglycol (techn.) | 3,00 |
| Methyl-4-hydoxybenzoat | 0,10 |
| Granuliertes Aminogel | 0,80 |
| ARP 100 | 5,0 |
| Ätznatronlauge (45%) | 7,50 |

Unter Erwärmen und Rühren wurden die aufgeführten Stoffe in Wasser gelöst. Daraus wird ein Gel mit einem pH-Wert von 3,8 und einer Dichte von 1,0 g/mL erhalten, die so verwendet werden kann.

Zur Überprüfung der Wirksamkeit dieser erfindungsgemäßen Mischung wurde diese einem Anwendungstest unterzogen. Dazu wurde die Mischung aus dem oben angegebenen Beispiel von 27 Testpersonen im Alter von 19 bis 55 Jahren über eine Zeitraum vom 6 Monaten auf einem ausgewählten Anwendungsareal angewandt. In 24 Fällen handelte es sich um ein Areal am Unterschenkel, in 2 Fällen um ein Areal am Oberschenkel und in einem Fall um ein Areal an der Achsel. Die Häufigkeit der Anwendung der Mischung betrug 1 bis 2 mal täglich. Zu Beginn der Studie sowie nach 3 Monaten und nach 6 Monaten fanden Befragungen der Testpersonen, eine Videoanalyse eines ausgewählten Testgebiets im Anwendungsareal und Untersuchungen auf eventuelle Nebenwirkungen und Unverträglichkeiten statt. Dieses Testgebiet wurde jeweils eine Woche vor Start der Behandlung sowie der Zwischenuntersuchungen rasiert. Während der Studie wurde zur Harrentfernung ausschließlich die Rasur durchgeführt.

Unmittelbar vor Beginn der Studie (Start), sowie nach 3 Monaten (3 M) und 6 Monaten (6 M) Anwendung wurde eine Befragung über die momentane Behaarung, das Haarwachstum, die Haarstärke und die Haardicke im Anwendungsareal durchgeführt.

Dabei wurde die momentane Behaarung auf einer Skala von 0, für keine Behaarung, bis 5, für sehr starke Behaarung, das Haarwachstum auf einer Skala von 0, für kein Wachstum, bis 2, für starkes Wachstum, die Haarstärke auf einer Skala von 0, für weich, bis 1, für hart, und die Haardicke auf einer Skala von 0, für dünn, bis 1, für dick bewertet. Die Ergebnisse sind in Tabelle 2 dargestellt.

**Tabelle 2:**

| | Behaarung | | | Haarwachstum | | | Haarstärke | | | Haardicke | | |
|---|---|---|---|---|---|---|---|---|---|---|---|---|
| Zeit | Start | 3 M | 6 M | Start | 3 M | 6 M | Start | 3 M | 6 M | Start | 3 M | 6 M |
| Mittelwert SD^{a} Median | 2,73 | 2,56 | 2,21 | 1,30 | 1,17 | 1,12 | 0,26 | 0,19 | 0,14 | 0,32 | 0,18 | 0,17 |
| | 0,77 | 0,82 | 0,90 | 0,45 | 0,37 | 0,27 | 0,38 | 0,37 | 0,31 | 0,42 | 0,36 | 0,35 |
| | 3 | 3 | 2 | 1 | 1 | 1 | 0 | 0 | 0 | 0 | 0 | 0 |
| Verb.(%)^{b} | | 37,0 | 48,1 | | 18,5 | 33,3 | | 22,2 | 18,5 | | 22,2 | 22,2 |

| | | | | | | | | | | | | |
|---|---|---|---|---|---|---|---|---|---|---|---|---|
| ^{a} SD = Standardabweichung | | | | | | | | | | | | |
| ^{b} Verb. (%) = prozentualer Anteil an Probanden, bei denen eine Verbesserung im jeweiligen Wert ermittelt wurde. | | | | | | | | | | | | |

Weiter wurde die subjektive Wirksamkeit der Mischung getestet. Dazu wurden die Probanden befragt, ob im Testzeitraum die Harre auf dem Testgebiet dünner geworden seien (dünnere Haare), ob das Haarwachstum sich verringet habe (geringeres Wachstum), ob die Haare leichter zu entfernen seien (leichter entfernbar) und ob die Haare weicher geworden seien (weichere Haare). Die Probanden hatten dabei die Möglichkeit zustimmend, unentschieden oder ablehnend zu antworten.

Die Ergebnisse der Befragung zur subjektiven Wirksamkeit sind in Tabelle 3 dargestellt:

**Tabelle 3:**

| | | dünnere Haare | geringeres Wachstum | leichter entfernbar | weichere Haare |
|---|---|---|---|---|---|
| 3 M | Zustimmung (%) | 50 | 54 | 50 | 50 |
| | unentschieden (%) | 25 | 21 | 29 | 29 |
| | Ablehnung (%) | 25 | 25 | 21 | 21 |
| 6M | Zustimmung (%) | 63 | 70 | 48 | 67 |
| | unentschieden (%) | 19 | 26 | 41 | 19 |
| | Ablehnung (%) | 19 | 4 | 11 | 15 |

Zur Videoanalyse wurde von jedem Probanden jeweils zum Start der Studie und nach 3 und 6 Monaten ein Videobild unter UV-Licht mit 480 auf 640 Bildpunkten von dem gleichen, ausgewählten Testgebiet der Haut mit einer Größe von 6 mm auf 8 mm angefertigt. Die Lage dieses Testgebiets wurde durch Vermessung der Abstände zu markanten Fixpunkten, wie Narben oder Verfärbungen, mit einer Genauigkeit von +- 3mm bestimmt. Anhand dieses Bilds wurde im Testareal jeweils die Anzahl der Haare und deren gemittelte Dicke in Bildpunkten bestimmt. Die Ergebnisse der Bildauswertung sind in Tabelle 4 aufgeführt:

**Tabelle 4**

| | Haarzahl | | | Haardicke (Bildpunkte) | | |
|---|---|---|---|---|---|---|
| | Start | 3 M | 6 M | Start | 3 M | 6 M |
| Mittelwert | 3,6 | 3,3 | 2,8 | 6,2 | 5,5 | 4,4 |
| SD | 1,6 | 2,1 | 1,3 | 2,0 | 1,8 | 1,9 |
| Median | 3,0 | 3,0 | 3,0 | 6,4 | 5,5 | 4,4 |
| Rückgang (%)^{c} | | 7,2 | 23,5 | | 10,6 | 28,9 |

| | | | | | | |
|---|---|---|---|---|---|---|
| ^{c}Rückgang (%) = Rückgang des Mittelwerts, bezogen auf den Startwert, angegeben in % | | | | | | |

Sowohl die subjektive Befragung der Probanden als auch die objektive Messung anhand der Videoaufnahmen bestätigen die Wirksamkeit der erfindungsgemäßen Mischung zur Verlangsamung und / oder Verhinderung von Haarwuchs.

Sowohl bei der Untersuchung nach 3 Monaten als auch bei der Untersuchung nach 6 Monaten waren bei den Probanden trotz täglicher Anwendung keine negativen Hautreaktionen auf die Mischung zu beobachten und alle entschlossen sich das Produkt weiter zu verwenden.

Damit konnte gezeigt werden, dass die erfindungsgemäße Mischung ein wirksames Produkt zur Verzögerung und / oder Verhinderung von Haarwuchs mit einer hohen Hautverträglichkeit darstellt.

## Patentansprüche

1. Verwendung einer Mischung zur Verlangsamung und/oder Verhinderung von Haarwuchs auf einer Hautpartie, auf der Haarwuchs auftritt, die, bezogen auf das Gesamtgewicht der Mischung:
(a) 5 bis 30 Gewichtsprozent Äpfelsäure,
(b) 1 bis 20 Gewichtsprozent eines Elektrolyten und
(c) 50 bis 94 Gewichtsprozent eines in der Kosmetik akzeptablen, auf Wasser basierenden Mittels, das 0,05 bis 40 Gewichtsprozent eines filmbildenden Mittels aufweist, bezogen auf das Gesamtgewicht des auf Wasser basierenden Mittels,
aufweist.

2. Verwendung nach Anspruch 1, **dadurch gekennzeichnet, dass** der Elektrolyt ausgewählt ist aus einem oder mehreren Mitgliedern der Gruppe, bestehend aus wasserlöslichen Salzen von Alkali- oder Erdalkalimetallen.

3. Verwendung nach Anspruch 1 oder 2, **dadurch gekennzeichnet, dass** der Elektrolyt Totes Meer Salz ist.

4. Verwendung nach Anspruch 1 bis 3, **dadurch gekennzeichnet, dass** der Anteil des Elektrolyten 5 bis 10 Gewichtsprozent des Gesamtgewichts der Mischung beträgt.

5. Verwendung nach Anspruch 1 bis 4, **dadurch gekennzeichnet, dass** das, in der Kosmetik akzeptable, auf Wasser basierende Mittel ausgewählt ist aus der Gruppe, bestehend aus Emulsionen, Mikroemulsionen und wässrigen Lösungen eines Filmbildenden Materials.

6. Verwendung nach Anspruch 1 bis 5, **dadurch gekennzeichnet, dass** das filmbildende Mittel ein oder mehrere Substanzen, ausgewählt aus der Gruppe, bestehend aus Hauterweichern, Emulgatoren, oberflächenaktiven Substanzen, Gelen, Feuchthaltemitteln, Verdickungsmitteln Pudern und anderen Proteinlösungen ist.

7. Verwendung nach Anspruch 1 bis 6, **dadurch gekennzeichnet, dass** die Mischung des Weiteren ein oder mehrere Antischweißmittel und /oder deodorierende Substanzen aufweist.

8. Verwendung nach Anspruch 1 bis Anspruch 7, **dadurch gekennzeichnet, dass** die Mischung des Weiteren ein Mittel zur Verzögerung des Wachstums von Körperhaaren aufweist.

9. Verwendung nach Anspruch 8, **dadurch gekennzeichnet, dass** das Mittel zur Verzögerung des Wachstums von Körperhaaren Aminosäuren aufweist, welche die Ausbildung der helicalen Struktur des Kreatins des Körperhaares destabilisieren oder zerstören.

10. Verwendung nach Anspruch 9, **dadurch gekennzeichnet, dass** die Aminosäure Prolin oder Hydroxyprolin ist.

11. Verwendung nach Anspruch 8 bis 10, **dadurch gekennzeichnet, dass** das Mittel zur Verzögerung des Wachstums von Körperhaaren antiandrogene Substanzen aufweist.

12. Verwendung nach Anspruch 11, **dadurch gekennzeichnet, dass** die antiandrogenen Substanzen ausgewählt sind aus Phytosterolen und makrozyklischen Tanninen.

13. Verwendung nach Anspruch 8 bis 12, **dadurch gekennzeichnet, dass** das Mittel zur Verzögerung des Wachstums von Körperhaaren aus Pflanzenstoffen extrahiert ist.

14. Verwendung nach Anspruch 13, **dadurch gekennzeichnet, dass** das Mittel zur Verzögerung des Wachstums von Körperhaaren aus der Sägepalme, aus Kürbiskernen und dem Weidenröschen extrahiert ist.

15. Verwendung nach Anspruch 1 bis 14, **dadurch gekennzeichnet, dass** der pH-Wert der Mischung nicht kleiner ist als 3,5 und nicht größer ist als 4,5.

16. Verwendung nach Anspruch 1 bis 15, **dadurch gekennzeichnet, dass** die Mischung eine Base zur Einstellung des pH-Werts aufweist.

17. Verwendung nach Anspruch 1 bis 16, **dadurch gekennzeichnet, dass** die Mischung in Form einer Creme, einer Lotion , eines Gels oder eines Sprays vorliegt.

18. Verwendung gemäß Anspruch 17, **dadurch gekennzeichnet, dass** die Hautpartie zuvor enthaart wurde.
